# EUROPÄISCHE PATENTSCHRIFT

(11) **EP 1 536 840 B1**
(45) Veröffentlichungstag und Bekanntmachung des Hinweises auf die Patenterteilung: **26.08.2009**
(21) Anmeldenummer: 03793597.0
(22) Anmeldetag: 02.09.2003
(51) Int. Cl.: A61K 9/08, A61K 9/72, A61K 47/02, A61K 47/24

(54) **FORMULIERUNG ZUR EINSCHLEUSUNG VON NUKLEINSÄUREN IN EUKARYOTISCHEN ZELLEN**
FORMULATION FOR INWARDLY TRANSFERRING NUCLEIC ACIDS INTO EUCARYOTIC CELLS
FORMULATION POUR INTRODUIRE DES ACIDES NUCLEIQUES DANS DES EUCARYOTES

(30) Priorität: 02.09.2002 DE 10240418
(43) Veröffentlichungstag der Anmeldung: 08.06.2005
(73) Patentinhaber: Avontec GmbH, 82152 Martinsried (DE)
(72) Erfinder: HECKER, Markus, 69118 Heidelberg (DE); WAGNER, Andreas, H., 69115 Heidelberg (DE)
(74) Vertreter: Dehmel, Albrecht
(86) Internationale Anmeldenummer: PCT/DE2003/002901
(87) Internationale Veröffentlichungsnummer: WO 2004/022102

(56) Entgegenhaltungen:
- WO-A-00/76554
- WO-A-01/88159
- WO-A-02/061037
- DE-A- 19 912 436
- US-A- 4 613 505
- US-B1- 6 323 029
- VAN SORGE A A ET AL: "Specificity of Flurbiprofen and Enantiomers for Inhibition of Prostaglandin Synthesis in Bovine Iris/Ciliary Body - Relationship between inhibition of thromboxane production and the plasma unbound concentration of S(+)-ibuprofen" PROSTAGLANDINS AND OTHER LIPID MEDIATORS, BUTTERWORTH, STONEHAM, MA, US, Bd. 55, Nr. 2-3, 1. Februar 1998 (1998-02-01), Seiten 169-177, XP004119455 ISSN: 0090-6980

## Beschreibung

Die vorliegende Erfindung betrifft eine pharmazeutische Formulierung umfassend Nukleinsäuren gemäß Anspruch 1.

Ein wesentliches Ziel der Dechiffrierung des menschlichen Genoms ist es, krankmachende Gene (aufgrund der Wirkungsweise ihrer Produkte) bzw. krankmachende Veränderungen in der Struktur dieser Gene (Polymorphismen) zu identifizieren und einem Krankheitsbild zuzuordnen. Damit scheint eine Kausaltherapie der meisten Erkrankungen in greifbare Nähe gerückt, vorausgesetzt dass diese durch eine definierte Anzahl von zu stark, zu schwach oder fehlerhaft exprimierten Genprodukten verursacht sind. In der Tat kennt man bereits für eine ganze Reihe von Erbkrankheiten (z.B. Mukoviszidose) den in der Regel singulären Gendefekt (monogenetische Erkrankung), während sich die Situation für andere Erkrankungen (z.B. Bluthochdruck) wesentlich komplexer darstellt. Diese Erkrankungen sind offenbar nicht das Resultat eines einzelnen, sondern multipler Gendefekte (polygenetische Erkrankung), welche die betröffenen Personen dazu prädestinieren, beim Zusammentreffen mit bestimmten Umweltfaktoren die Erkrankung zu entwickeln. Ungeachtet dieser Einschränkung bietet der gezielte Eingriff in die Expression eines oder mehrerer Gene die Chance einer ursachen- und nicht lediglich symptombezogenen Therapie.

Für diese "Gentherapie" gibt es nach dem derzeitigen Stand der Technik vier Möglichkeiten. So ist es heute ohne weiteres möglich, mit Hilfe einer Genfähre ein Ersatz-Gen in Körperzellen einzuschleusen und es dort von der zelleigenen Proteinsynthese-Maschinerie in das entsprechende Protein umschreiben zu lassen (liposomaler Transfer eines Plasmid, transiente Expression) bzw. dieses in das Genom der Zielzellen zu integrieren (viraler Gentransfer, stabile Expression). Große Schwierigkeiten gibt es aber noch bei der korrekten Adressierung der Zielzellen, der Transfereffizienz und, soweit erforderlich, dem An- bzw. Abschalten des transferierten Gens. Darüber hinaus haben die zurzeit verwendeten liposomalen bzw. viralen Transfersysteme häufig einen zellschädigenden Effekt oder lösen eine unter Umständen dramatische, immunologisch bedingte Unverträglichkeitsreaktion aus.

Um im Gegensatz zum Gentransfer die Expression eines krankmachenden Gens zu verhindern, gibt es zur Zeit erstens die Möglichkeit, diese während der Übersetzung der Boten-RNA (mRNA) in das entsprechende Protein, der so genannten Translation, spezifisch zu unterbinden. Bei dieser Antisense-Technik werden kurze (in der Regel 15-25 Nukleotide umfassende) DNA-Einzelstränge in die Zielzellen eingeschleust, die eine für ihre Ziel-mRNA komplementäre Basenfolge (Sequenz) aufweisen. Durch Anlagerung des Antisense-Oligonukleotids an die ebenfalls einzelsträngige mRNA (DNA-RNA-Hybridbildung) kommt es zum Abbruch der Translation. Bei der zweiten derartigen Möglichkeit, der so genannten RNA-Interferenz (RNAi), wird dagegen ein genau 21 Basenpaare umfassender RNA-Doppelstrang in die Zelle eingeschleust, dessen Sequenz identisch mit einem Abschnitt der für das Zielprotein kodierenden mRNA ist. In der Folge wird in der Zielzelle ein noch nicht im Detail bekannter Komplex von Proteinen gebildet, der spezifisch die Ziel-mRNA spaltet und somit deren Translation verhindert. Beiden Techniken gemeinsam ist ein Problem: Die DNA-Einzelstränge bzw. RNA-Doppelstränge scheinen nicht von alleine in die Zielzellen aufgenommen werden, sondern müssen wie die wesentlich größeren Plasmide (in der Regel mehrere Tausend Basenpaare groß) in diese transfiziert werden. Dazu werden sie in der Regel in Liposomen verpackt, die als Transportmittel dienen.

Bei dem dritten Verfahren zum gezielten Eingriff in die Genexpression werden kurze DNA-Doppelstränge eingesetzt, so genannte Decoy-Oligonukleotide. Der erste Schritt bei der Expression eines Gens ist das Übersetzen des entsprechenden DNA-Abschnitts auf dem Chromosom in einen RNA-Einzelstrang, die Transkription. Entscheidend für die Initiation der Transkription sind die so genannten Transkriptionsfaktoren. Diese regulatorischen Proteine binden an die Starterregion des Gens (Promotorregion) und leiten die Transkription des Gens durch die RNA-Polymerase ein. Darüber hinaus können an die DNA gebundene Transkriptionsfaktoren diesen Übersetzungsvorgang aber auch blockieren. Decoy-Oligonukleotide sind kurze (in der Regel 15-25 Basenpaare umfassende) DNA-Doppelstränge, die das Sequenzmotiv imitieren, an welches sich der Ziel-Transkriptionsfaktor in der Starterregion seines (seiner) Zielgens (Zielgene) bindet. Jeder Transkriptionsfaktor erkennt nur das für ihn passende Sequenzmotiv, dadurch ist der Decoy-Oligonukleotid-Ansatz spezifisch.

Folge der durch das Decoy-Oligonukleotid im Zytoplasma oder im Zellkern stattfindenden Neutralisierung des Transkriptionsfaktors ist, dass dieser nicht mehr die Expression seines (seiner) Zielgens (Zielgene) induzieren bzw. blockieren kann.
US 4523029 beschreibt die verbesderte Absorption von Oliganucleotiden über die Mucose nach Zugabe von Penetrations - vermittlern wie nicht steroidalen Antiphlogistika.
Es besteht somit der dringende Bedarf nach einem einfachen und für die Zellen und den Organismus nicht belastenden Mittel für die Einschleusung von Nukleinsäuren.

### Die Aufgabe wird durch den in den Patentansprüchen definierten Gegenstand gelöst

Die Erfindung wird durch die nachfolgenden Figuren näher erläutert:

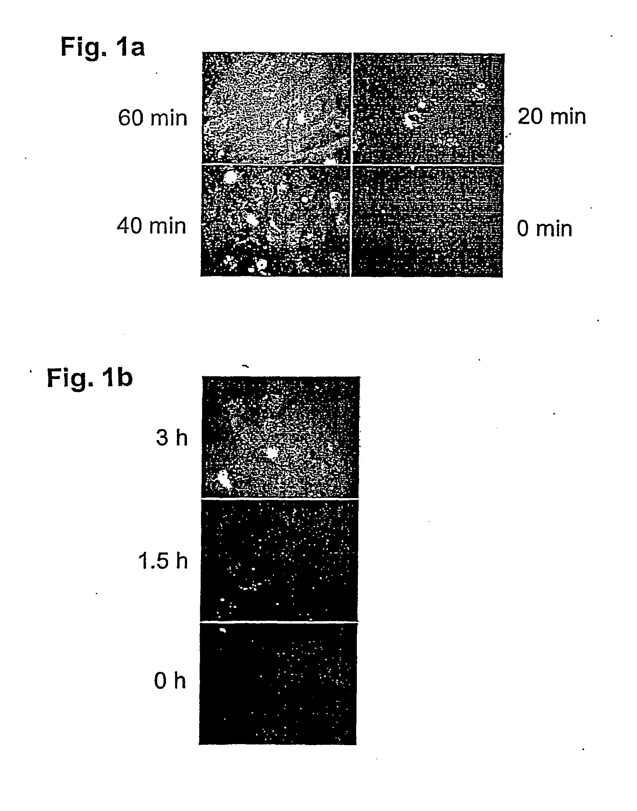

Figur 1 zeigt exemplarisch das Ergebnis der zeitabhängigen Aufnahme eines FITC-markierten Decoy-Oligonukleotids gegen den Transkriptionsfaktor C/EBP (10 µmol/l; Fig 1 a) und eines FITC-markierten Decoy-Oligonukleotids gegen den Transkriptionsfaktor AP-1 (10 µmol/l; Fig. 1 b) in humane kultivierte Endothelzellen, die in Zellkulturmedium inkubiert worden waren. Die Aufnahme der Fluoreszenzfarbstoff-markierten Nukleinsäuren wurde mittels Fluoreszenz-mikroskopie nachgewiesen (Vergrößerung 400x).

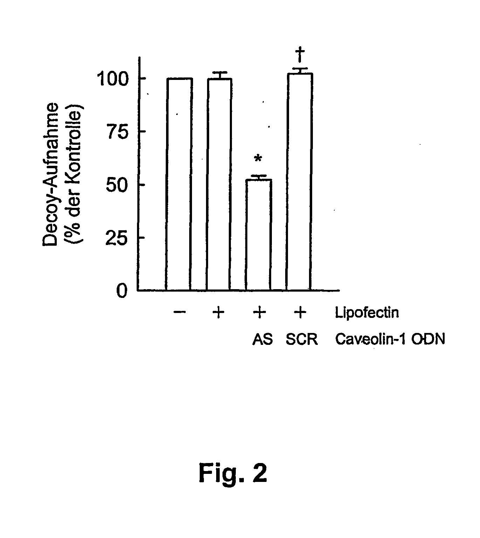

Figur 2 zeigt in einem Balkendiagramm den Effekt einer Antisense-Oligonukleotid (AS)-gestützten Reduktion der Proteinexpression von Caveolin-1 (37±10% der Kontrolle, n=3) in humanen kultivierten Endothelzellen auf die Aufnahme des FITC-markierten C/EBP-Decoy-Oligonukleotids (10 µmol/l) über einen Zeitraum von 1 h. Statistische Zusammenfassung (n=3-4, bezogen in % auf die Aufnahme des Decoy-Oligonukleotids in unbehandelten Kontrollzellen; *P<0,05 versus Kontrolle, †P<0,05 versus AS). SCR (scrambled) steht für die Behandlung der Endothelzellen mit einem Oligonukleotid gleicher Basenzusammensetzung aber unterschiedlicher Sequenz im Vergleich mit dem Antisense-Oligonukleotid.

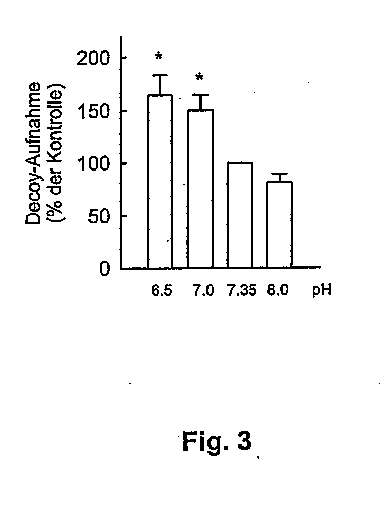

Figur 3 zeigt in einem Balkendiagramm den Effekt einer Veränderung des extrazellulären pH-Wertes auf die Aufnahme des FITC-markierten C/EBP-Decoy-Oligonukleotids (10 µmol/l) in humanen kultivierten Endothelzellen über einen Zeitraum von 1 h. Statistische Zusammenfassung (n=4-5, bezogen in % auf die Aufnahme des Decoy-Oligonukleotids bei pH 7,35; *P<0,05).

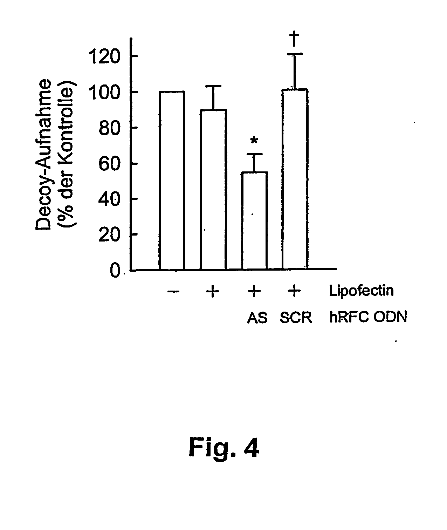

Figur 4 zeigt in einem Balkendiagramm den Effekt einer Antisense-Oligonukleotid (AS)-gestützten Reduktion der Proteinexpression des reduzierten Folsäure-Carriers (auf 33±10% der Kontrolle, n=5) in humanen kultivierten Endothelzellen auf die Aufnahme des FITC-markierten C/EBP-Decoy-Oligonukleotids (10 µmol/l) über einen Zeitraum von 1 h. Statistische Zusammenfassung (n=3, bezogen in %, auf die Aufnahme des Decoy-Oligonukleotids in unbehandelten Kontrollzellen; *P<0,05 versus Kontrolle, ^{†}P<0,05 versus AS). SCR (*scrambled*) steht für die Behandlung der Endothelzellen mit einem unwirksamen Antisense-Oligonukleotid.

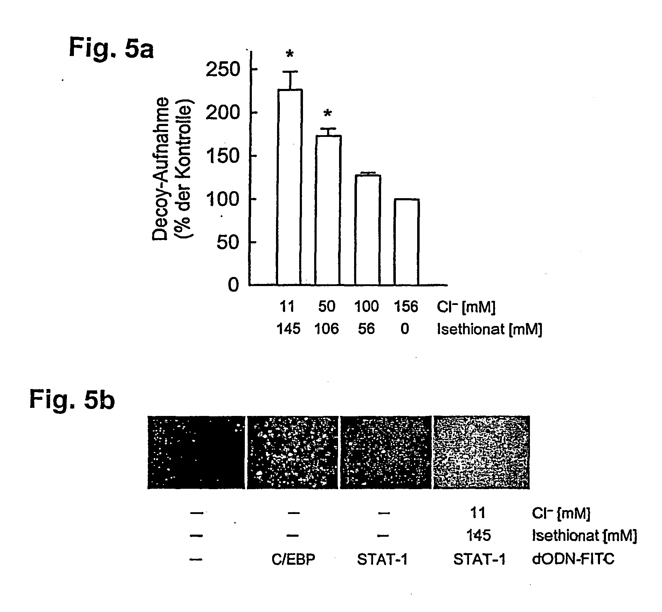

Figur 5 zeigt in einem Balkendiagramm (a) den Effekt einer Veränderung der extrazellulären Chloridionen-Konzentration (schrittweiser Ersatz durch Isethionat) auf die Aufnahme des FITC-markierten C/EBP-Decoy-Oligonukleotids (10 µmol/l) in humanen kultivierten Endothelzellen über einen Zeitraum von 1 h. Statistische Zusammenfassung (n=4, bezogen in % auf die Aufnahme des Decoy-Oligonukleotids bei 156 mmol/l Cl⁻; *P<0,05). Figur (b) zeigt exemplarisch den Effekt einer Absenkung der extrazellulären Chlorid-Konzentration von 156 auf 11 mmol/l auf die Aufnahme eines FITC-markierten STAT-1-Decoy-Oligonukleotids in humanen kultivierten Endothelzellen über einen Zeitraum von 1 h (fluoreszenzmikroskopische Aufnahmen, Vergrößerung 200x).

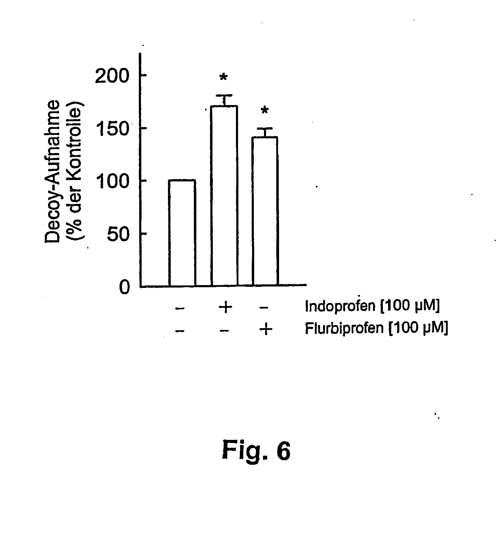

Figur 6 zeigt in einem Balkendiagramm den Effekt einer Ko-Inkubation mit Flurbiprofen oder Indoprofen (jeweils 100 µmol/l) auf die Aufnahme des FITC-markierten C/EBP-Decoy-Oligonukleotids (10 µmol/l) in humanen kultivierten Endothelzellen über einen Zeitraum von 1 h. Statistische Zusammenfassung (n=4, bezogen in % auf die Aufnahme des Decoy-Oligonukleotids in unbehandelten Kontrollzellen; *P<0,05).

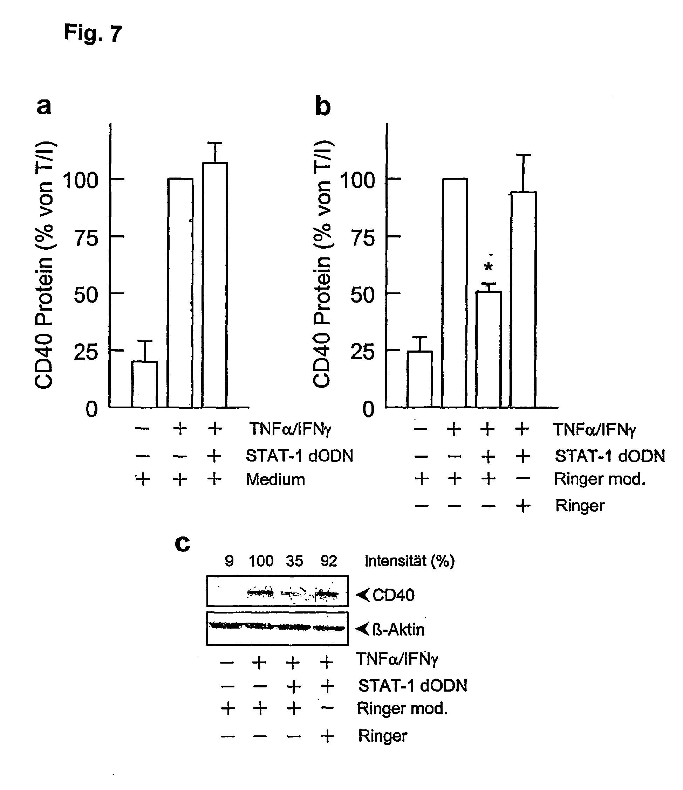

Figur 7 zeigt in Balkendiagrammen (a, b) und in einer repräsentativen Western Blot-Analyse (c) den Effekt von (a) Zellkulturmedium (n=3) und (b, c) unmodifizierter bzw. modifizierter (mod.) Ringerlösung (11 mmol/l Chloridionen, pH 7,0) als Inkubationsmedium auf die STAT-1 Decoy-Oligonukleotid-vermittelte Hemmung der zytokin-stimulierten (100 U/ml Tumomekrosefaktor α [TNFα] plus 1000 U/ml Interferon-γ [IFNγ] für 10 h) CD40-Proteinexpression in humanen kultivierten Endothelzellen (bezogen in % auf die Proteinmenge in zytokin-stimulierten Zellen [T/I]); *P<0,05 versus T/I; b, statistische Zusammenfassung, n=6; c, repräsentative Western Blot-Analyse mit β-Aktin als internem Standard). Die Endothelzellen wurden mit den unmarkierten Decoy-Oligonukleotiden (10 µmol/l) für 30 min vor der Zytokin-Exposition vorinkubiert.

Im Gegensatz zu Plasmiden, Antisense- und RNAi-Oligonukleotiden können Decoy-Oligonukleotide (doppelsträngige DNA-Oligonukleotide) offenbar auch ohne Hilfsmittel (Transfektionsreagenz) in die jeweilige Zielzelle gelangen. Über den diesem Transport zugrunde liegenden Mechanismus war bis dato nichts bekannt. Den Erfindern ist es gelungen, diesen Mechanismus aufzuklären. Auf der Basis der hierbei gewonnenen Erkenntnisse werden neue Formulierungen für die Applikation von Nukleinsäuren in eukaryotische Zellen, besonders in Säügetierzellen und insbesondere in humane Zellen bereitgestellt. -

Der Begriff "Formulierung" oder "pharmazeutische Formulierung" wie hier verwendet bezeichnet die pharmazeutische Zubereitungsform für z.B. ein Arzneimittel oder einen Impfstoff, die einem Menschen, einem Tier *in vivo* oder Organen, Geweben oder Zellen *in vitro* oder *ex vivo* verabreicht wird, bestehend aus einem oder mehreren Wirkstoffen und Formulierungshilfsmitteln. Wirkstoffe sind gemäß der vorliegenden Erfindung Nukleinsäuren.

Der Begriff "Formulierungshilfsmittel" wie hier verwendet bezeichnet alle Bestandteile der vorstehend erwähnten pharmazeutische Zubereitungsform mit Ausnahme der Wirkstoffe. Formulierungshilfsmittel können z.B. physiologische Salz- oder Pufferlösungen, Wasser, Konservierungsmittel, Ionen, Säuren, Basen, Konservierungslösungen für die Organtransplantation, Blutersatzflüssigkeiten, Inhalations-, Infusions- und Injektionslösungen sowie Medikamente sein.

Die vorliegende Erfindung betrifft eine neue Formulierung zur Einschleusung von Nukleinsäuren in eukaryotische Zellen, **dadurch gekennzeichnet, dass** die Formulierung einen pH-Wert im Bereich von etwa pH 6,0 bis etwa pH 7,4, vorzugsweise im Bereich von etwa pH 6,2 bis etwa pH 7,0 und besonders bevorzugt von etwa pH 6,5 oder 7,0 aufweist und/oder eine Anionen-Konzentration vorzugsweise eine Chloridionen-Konzentration im Bereich von etwa 5 bis etwa 100 mmol/l, vorzugsweise im Bereich von etwa 5 bis etwa 50 mmol/l und besonders bevorzugt im Bereich von etwa 5 bis etwa 10 mmol/l aufweist und/oder nichtsteroidale Antiphlogistika, z.B. Flurbiprofen oder Indoprofen, in einer Konzentration im Bereich von etwa 10 bis etwa 500 µmol/l, vorzugsweise im Bereich von etwa 50 bis etwa 250 µmol/l und besonders bevorzugt in einer Konzentration von etwa 100 µmol/l aufweist. Ferner kann die Formulierung neben den Wirkstoffen und den vorstehend beschriehenen Merkmalen noch ein oder mehrere geeignete Puffer umfassen. Ein Beispiel eines solchen Puffers ist eine modifizierte Ringer-Lösung enthaltend 145 mmol/l Na⁺, 5 mmol/l K⁺, 11 mmol/l Cl⁻, 2 mmol/l Ca²⁺, 1 mmol/l Mg²⁺, 10 mmol/l Hepes, 145 mmol/l Isethionat, 10 mmol/l D-Glucose, wobei der pH-Wert im Bereich von 6,5 bis 7,0 vorzugsweise bei 6,5 oder 7,0 liegt.

Zunächst konnten die Erfinder beobachten, dass die intrazelluläre Verteilung der von den untersuchten humanen Endothelzellen aufgenommenen Fluoreszenzfarbstoff-markierten Decoy-Oligonukleotide heterogen ist. Neben Ansammlungen in Vesikel-artigen Strukturen zeigte sich eine stärker diffuse Markierung von Zytoplasma und Zellkern. Insbesondere die Konzentrierung der Nukleinsäuren in Vesikeln gab Anlass zu der Vermutung, dass es sich bei dem Aufnahmeprozess um einen Rezeptor-vermittelten Endozytose-artigen Prozess handeln könnte.

Im Weiteren zeigte sich, dass humane Endothelzellen ebenso wie glatte Gefäßmuskelzellen oder Monozyten einen oder beide Varianten des Folsäure-Rezeptors exprimieren, einem potenziellen Kandidaten für die Aufnahme der Nukleinsäuren in diese Zellen. Dieser Rezeptor ist vorzugsweise in Einstülpungen der Zellmembran, den so genannten Caveolae lokalisiert und Destruierung der Caveolae - durch Entzug von Cholesterin oder Hemmung der Expression von Caveolin-1 (Figur 2) - führte zu einer deutlichen Einschränkung der Decoy-Oligonukleotid-Aufnahme.

Absenkung des extrazellulären pH-Wertes begünstigt dagegen die Rezeptor-vermittelte Folsäurebindung (Affinität), und auch für die Aufnahme der Decoy-Oligonukleotide in die humanen Endothelzellen konnte diese pH-Abhängigkeit gezeigt werden (Figur 3). Nach Bindung der Folsäure an den Rezeptor werden die Caveolae internalisiert (Potozytose; RGW Anderson (1998) Annu. Rev. Biochem. 67, 199). Um die in diesen Vesikeln eingeschlossene Folsäure in das Zytoplasma freizusetzen, wird ein Anionen-Transporter Carrier) benötigt, der durch Probenecid gehemmt werden kann (Kamen et al. (1991) J. Clin. Invest. 87, 1442) und nicht identisch mit dem weiter unten beschriebenen reduzierten Folsäure-Carrier hFRC ist. Tatsächlich war auch die Akkumulation der Decoy-Oligonukleotide in den humanen Endothelzellen Probenecid-sensitiv.

Die erfindungsgemäße Formulierung betrifft somit eine Formulierung mit einem pH-Wert im Bereich von etwa pH 6,0 bis etwa pH 7,4. Vorzugsweise liegt der pH-Wert im Bereich von etwa pH 6,2 bis etwa pH 7,0 und besonders bevorzugt bei etwa pH 6,5 oder 7,0.

Neben der Rezeptor-vermittelten Potozytose ist der primäre Transportweg für Folsäure in Säugetierzellen die Aufnahme über den reduzierten Folsäure-Carrier hRFC (LH Matherly (2001) Prog. Nucleic Acid Res. Mol. Biol. 67, 131). Im Prinzip sollte jede Körperzelle, die zur Zellteilung befähigt ist, über diesen Transporter verfügen, da Folsäure essentiell für die DNA-Synthese ist (siehe auch Whetstine et al. (2002) Biochem. J. Jul 29 [epub ahead ofprint]). Auch humane Endothelzellen exprimieren den hFRC. Die Antisense-Oligonukleotid-gestützte Reduktion der Expression des hRFC-Proteins auf ein Drittel in diesen Zellen hatte eine Hemmung der Decoy-Oligonukleotid-Aufnahme um 45% zur Folge (Abbildung 4). Weitere Hinweise auf die Beteiligung dieses Transportsystems an der Decoy-Oligonukleotid-Aufnahme (siehe Charakteristika des hFRC beschrieben in LH Matherly (2001) Prog. Nucleic Acid Res. Mol. Biol. 67, 131) waren deren wesentlich bessere Hemmung durch das Antifolat Methotrexat im Vergleich zu Folsäure sowie die hohe Empfindlichkeit gegenüber dem Anionenaustauscher-Inhibitor DIDS (4,4'-Diisothiocyano-2,2'-stilben-disulfonsäure).

Tatsächlich ist es für die hRFC-vermittelte Aufnahme des Anions Folsäure in Säugetierzellen notwendig, dass im Gegenzug ein Anion, vorzugsweise Chlorid die Zelle verlässt (Antiport) bzw. ein Kation, vorzugsweise ein Proton (H⁺) mit in die Zelle transportiert wird (Symport). Da der Carrier aber die höchste Affinität für Folsäure bzw. Methotrexat bei einem quasi physiologischen pH-Wert von 7,5 hat, verfehlt eine Absenkung des extrazellulären pH (d.h., ein Anstieg der Protonenkonzentration) den gewünschten Effekt einer verbesserten Nukleinsäureaufnahme über diesen Transportweg. Vielversprechender ist eine Erleichterung des Chloridtransports aus der Zelle, z.B. durch Reduktion der extrazellulären Chloridkonzentration (typischerweise 120 mmol/l) vorzugsweise unter den intrazellulären Wert (12 mmol/l), so dass ein nach Außen gerichteter Konzentrationsgradient für Chlorid entsteht. Wie in Figur 5 gezeigt, führte die Reduktion der extrazellulären Chloridkonzentration in der Tat zu einer signifikanten Verbesserung der Decoy-Oligonukleotid-Aufnahme in die humanen Endothelzellen.

Zusammengefasst belegen die vorgenannten Befunde, dass neben der pH-sensitiven Folatrezeptor-vermittelten Potozytose die Aufnahme von Nukleinsäuren in humane Zellen über den reduzierten Folsäure-Carrier erfolgt und sich die Effizienz dieses Transportwegs durch Absenkung der extrazellulären Anionen- insbesondere der Chloridkonzentration wesentlich steigern lässt.

Die erfindungsgemäße Formulierung betrifft somit eine Formulierung, umfassend eine Anionen-Konzentration vorzugsweise eine Chloridionen-Konzentration im Bereich von etwa 5 bis etwa 100 mmol/l, vorzugsweise im Bereich von etwa 5 bis etwa 50 mmol/l und besonders bevorzugt im Bereich von etwa 5 bis etwa 10 mmol/l. Ferner kann der physiologische Ersatz von Chloridionen beispielsweise durch den Zusatz einer äquimolaren Menge von Isethionat erfolgen.

Neben der Aufnahme spielt auch die Ausschleusung einer Substanz eine wichtige Rolle für deren aktuelle Konzentration bzw. Verfügbarkeit in der Zelle. Für Folsäure ist ein derartiger Transportweg aus Säugetierzellen beschrieben worden, der durch entzündungshemmende Medikamente (nichtsteroidale Antiphlogistika) wie Flurbiprofen oder Indoprofen hemmbar ist (M Saxena, GB Henderson (1996) Biochem. Pharmacol. 51, 974). Wie Figur 6 zeigt, werden auch Decoy-Oligonukleotide über diesen Transportweg aus humanen Zellen entfernt, d.h. die Konzentration der Nukleinsäuren in der Zelle kann durch Blockade dieses Transportwegs signifikant erhöht werden.

Die erfindungsgemäße Formulierung betrifft somit eine Formulierung, umfassend nichtsteroidale Antiphlogistika wie z.B. Flurbiprofen oder Indoprofen in einer Konzentration im Bereich von etwa 10 bis etwa 500 µmol/l, vorzugsweise im Bereich von etwa 50 bis etwa 250 µmol/l und besonders bevorzugt in einer Konzentration von etwa 100 µmol/l.

Die vorstehend beschriebenen Transportwege können neben Decoy-Oligoukleotiden auch von anderen Nukleinsäuren, z.B. von einzelsträngigen RNA/DNA-Oligonukleotiden oder von doppelsträngigen RNA-Oligonukleotiden in einem vergleichbaren Umfang genutzt werden und sind nicht auf Endothelzellen beschränkt. So wurden FITC-markierte einzelsträngige DNA-Oligonukleotide genauso effektiv in humane Endothelzellen transportiert wie die entsprechenden doppelsträngigen (Decoy-)Oligonukleotide, und die Geschwindigkeit der Aufnahme von Decoy-Oligonukleotiden in humane Endothel- und glatte Gefäßmuskelzellen war weitgehend identisch.

Abgesehen von der grundsätzlichen Voraussetzung, dass, beispielsweise Decoy-Oligonukleotide ihren Ziel-Transkriptionsfaktor effektiv neutralisieren, ist es für therapeutische Wirksamkeit von Nukleinsäuren entscheidend, dass sie schnell und in einem ausreichenden Umfang in die Zielzelle aufgenommen werden ohne auf potenziell zytotoxische Hilfsmittel zurückgreifen zu müssen. Insofern sind Verfahren der vorliegenden Erfindung zur Applikation dieser Nukleinsäuren die Verwendung geeigneter Puffer, umfassend
1. einen pH-Wert im Bereich von etwa pH 6,0 bis pH 7,4, vorzugsweise im Bereich von etwa pH 6,2 bis pH 7,0 und besonders bevorzugt bei etwa pH 6,5 oder 7,0, und/oder
2. eine extrazelluläre Anionenkonzentration, vorzugsweise eine Chloridkonzentration (z.B. durch Zusatz von Isethionat) im Bereich von etwa 5 bis etwa 100 mmol/l, vorzugsweise im Bereich von etwa 5 bis etwa 50 mmol/l und besonders bevorzugt im Bereich von etwa 5 bis etwa 10 mmol/l, und/oder
3. nichtsteroidale Antiphlogistika, vorzugsweise Flurbiprofen oder Indoprofen, mit einer Konzentration im Bereich von etwa 10 bis etwa 500 µmol/l, vorzugsweise im Bereich von etwa 50 bis etwa 250 µmol/l und besonders bevorzugt mit einer Konzentration von etwa 100 µmol/l.

Die vorliegende Erfindung betrifft ferner eine Formulierung zur Einschleusung von Nukleinsäuren in eukaryotische Zellen, wobei zwei oder alle der vorgenannten Merkmale kombiniert werden können. Ein Beispiel für die dadurch hervorgerufene, größere biologische Wirksamkeit der Nukleinsäuren zeigt Figur 7. Eine bevorzugte Formulierung umfasst eine Kombination der erfindungsgemäßen pH-Wert und Chloridkonzentration- Einstellung.

Eine mit den Zielzellen in Berührung gebrachte Formulierung gemäß der Erfindung enthält in einer bevorzugten Ausführungsform nur Nukleinsäure (in einer Konzentration von 0,01 bis 100 µmol/l) und Puffer. Es können ein oder mehrere geeignete Puffer zugesetzt werden. Ein Beispiel eines solchen Puffers ist eine modifizierte Ringer-Lösung enthaltend 145 mmol/l Na⁺, 5 mmol/l K⁺, 11 mmol/l Cl⁻, 2 mmol/l Ca²⁺, 1 mmol/l Mg²⁺, 10 mmol/l Hepes, 145 mmol/l Isethionat, 10 mmol/l D-Glucose, pH 6,5 oder pH 7,0, wie sie in dem in Figur 7 gezeigten Experiment verwendet worden ist.

Die Formulierung, die in dem Verfahren der gegenwärtigen Erfindung eingesetzt wird, wird bevorzugt lokal angewendet durch Injektion, Infusion, Inhalation, oder jede andere Applikationsform, die einen lokalen Zugang ermöglicht. Auch die *ex vivo* Anwendung der Formulierung (Inkubation von Blutgefäßen, Gewebe oder Zellen), verwendet im Verfahren der gegenwärtigen Erfindung, erlaubt einen lokalen Zugang. Ziel ist es, die Nukleinsäure-haltige Mischung möglichst nahe an die zu behandelnden Zellen heran zu bringen und damit - zumindest kurzzeitig - ein für die Aufnahme der Nukleinsäuren in die Zielzellen optimales extrazelluläres Milieu zu schaffen.

Die folgenden Beispiele dienen nur der Erläuterung und beschränken in keiner Weise den Umfang der Erfindung.

### 1. Zellkultur

Humane Endothelzellen wurden durch Behandlung mit 1,6 U/ml Dispase in Hepes-modifizierter Tyrodelösung für 30 Min. bei 37°C aus Nabelschnurvenen isoliert und auf Gelatine-beschichteten 6-Loch-Gewebekulturschalen (2 mg/ml Gelatine in 0,1 M HCl für 30 Min. bei Umgebungstemperatur) in 1,5 ml M199 Medium (Gibco Life Technologies, Karlsruhe, Deutschland), enthaltend 20% fötales Kälberserum, 50 U/ml Penicillin, 50 µg/ml Streptomycin, 10 U/ml Nystatin, 5 mM HEPES und 5 mM TES, 1 µg/ml Heparin und 40 µg/ml endothelialer Wachstumsfaktor, kultiviert. Sie wurden durch ihre typische Pflasterstein-Morphologie, positive Immunfärbung für von Willebrandt-Faktor (vWF) und fluorimetrischen Nachweis (FACS) von PECAM-1 (CD31) sowie negative Immunfärbung für glattmuskuläres α-Actin (Krzesz et al. (1999) FEBS Lett. 453, 191) identifiziert.

Humane glatte Gefäßmuskelzellen wurden aus den Venen excidierter Thymusdrüsen isoliert. Nach Entfernung von adhärentem Binde- und Fettgewebe wurde das Gefäß mit Hilfe eines Skalpells mechanisch zerkleinert. Im Anschluss daran erfolgte eine Inkubation bei 37°C und 5 % CO₂ für 14-16 Stunden in einer Verdauungslösung (5% fetales Rinderserum, 5 mmol/l HEPES, 5 mmol/l TES, 50 U/ml Penicillin, 50 µg/ml Streptomycin, 10 U/ml Nystatin und 0,15% Kollagenase (Clostridium histolyticum, Sigma-Aldrich, Deisenhofen) in DMEM Medium; Gibco Life Technologies). Nach Zentrifugation der Zellsuspension bei 1.000 rpm für 5 min bei Raumtemperatur wurde das Zellpellet in 2-3 ml Kultivierungsmedium (Smooth Muscle Cell Growth Medium 2, PomoCell GmbH, Heidelberg) suspendiert und in Gewebekulturschalen ausplattiert, die zuvor mit Gelatine (2 mg Gelatine pro ml 0,1 N HCL) für mindestens 30 min bei Raumtemperatur beschichtet und anschließend zweimalig mit Medium gewaschen wurden. Das Kultivierungsmedium wurden unter sterilen Bedingungen nach 2 Tagen ausgetauscht und die Zellen kurz mit Medium gewaschen. Im Folgenden wurde alle 4 Tage das Medium gewechselt.

Die humane Monozyten-Zelllinie THP-1 (ATCC TIB 202) wurde in RPMI 1640 Medium (Gibco Life Technologies), enthaltend 10% fötales Kälberserum, 50 U/ml Penicillin, 50 µg/ml Streptomycin und 10 U/ml Nystatin, kultiviert.

### 2. Decoy-Oligonukleotid-Synthese

Doppelsträngige Decoy-Oligonukleotide wurden von den komplementären einzelsträngigen, Fluoresceinisothiocyanat (FITC)-markierten Oligonukleotiden (Eurogentec, Köln, Deutschland) wie bei Krzesz et al. (1999) FEBS Lett. 453, 191 beschrieben hergestellt. Die einzelsträngigen Sequenzen der Oligonukleotide waren wie folgt (unterstrichene Buchstaben kennzeichnen Phosphorothioat-verbundene Basen):
- AP-1,: 5'-CGCTTGATGACTCAGCCGGAA-3' (SEQ ID NO:1)
- C/EBP,: 5'-TGCAGATTGCGCAATCTGCA-3' (SEQ ID NO:2)
- STAT-1,: 5'-CATGTTATGCATATTCCTGTAAGTG-3' (SEQ ID NO:3)

### 3. Antisense-Oligonukleotid-Synthese und Inkubation

Für einen Antisense-Ansatz wurde 1 ml Kulturmedium mit 3% Lipofectin (v/v) (Gibco Life Technologies) versetzt und für 60 Minuten bei Raumtemperatur (RT) inkubiert. Im Anschluss daran wurde das entsprechende Antisense- oder Kontroll-Oligonukleotid (Eurogentec, Köln, Deutschland) in einer finalen Konzentration von 0,5 µmol/l hinzugegeben und weitere 30 Minuten bei Raumtemperatur inkubiert. Bei Versuchsbeginn wurden die entsprechenden Mengen Heparin und endothelialer Wachstumsfaktor hinzugefügt und das herkömmliche Zellkulturmedium der Endothelzellkultur durch das Antisense-Lipofectin-Medium ersetzt. Nach 6 Stunden wurde das Antisense-Lipofectin-Medium entfernt und durch frisches Zellkulturmedium ersetzt; 24 Stunden nach der Transfektion erfolgte dann die Western Blot-Analyse bzw. die fluoreszenzmikroskopische Analyse der Decoy-Oligonukleotid-Aufnahme.

Die Sequenz (Phosphorthioesterbindungen sind mit * markiert) des Antisense-Oligonukleotids für Caveolin-1 war 5'-A*T*G*TCCCTCCGAGT*C*T*A-3' (SEQ ID NO:4), als Kontrolle wurde ein *scrambled* Oligonukleotid mit identischer Basenzusammensetzung wie das Antisense-Oligonukleotid aber unterschiedlicher Sequenz (5'-C*T*C*GATCCTGACTA*C*T*G-3') (SEQ ID NO:5) verwendet. Die Sequenz des Antisense-Oligonukleotids für den reduzierten Folatcarrier (hRFC) war 5'-C*A*A*A*GG*T*A*GC*A*C*A*CG*A*G-3' (SEQ ID NO:6). Auch hier wurde als Kontrolle ein *scrambled* Oligonukleotid eingesetzt (5'-A*C*A*T*GG*A*C*A*CG*A*A*GC*A*G-3') (SEQ ID NO:7).

### 4. RT-PCR-Analyse

Die zelluläre Gesamt-RNA wurde mit dem Qiagen RNeasy Kit (Qiagen, Hilden, Deutschland) isoliert, daran anschließend wurde eine cDNA-Synthese mit einem Maximum von 3 µg RNA und 200 U Superscript ^{™} II Reversetranskriptase (Gibco Life Technologies) in einem Gesamtvolumen von 20 µl entsprechend der Herstelleranleitung durchgeführt. Für die anschließende Polymerasekettenreaktion wurden 5 µl der cDNA-Lösung und 1 U Taq DNA-Polymerase (Gibco Life Technologies) in einem Gesamtvolumen von 50 µl benutzt. Die PCR-Produkte wurden auf 1,5% Agarose-Gelen enthaltend 0,1% Ethidiumbromid separiert und die Intensität der Banden wurde densitometrisch mit einem CCD-Kamerasystem und der One-Dscan Gelanalyse-Soflware von Scanalytics (Billerica, MA, USA) dokumentiert.

Alle PCR-Reaktionen wurden einzeln für jedes Primer-Paar in einem *Tpersonal* Cycler (Biometra, Göttingen, Deutschland) durchgeführt:
hFR1 (Folatrezeptor α), Produktgröße 181 bp, 37 Zyklen, Anlagerungstemperatur 60°C, (Vorwärtsprimer), 5'-CAAGGTCAGCAACTACAGCCGAGGG-3' (SEQ ID NO:8), (umgekehrter Primer) 5'-TGAGCAGCCACAGCAGCATTAGGG-3' (SEQ ID NO:9).
hFR2 (Folatrezeptor β), Produktgröße 385 bp, 37 Zyklen, Anlagerungstemperatur 61°C, (Vorwärtsprimer) 5'-CTGTGTAGCCACCATGTGCAGTGC-3' (SEQ ID NO:10), (umgekehrter Primer) 5'-TGTGACAATCCTCCCACCAGCG-3') (SEQ ID NO:11).
h1RFC, Produktgröße 333 bp, 37 Zyklen, Anlagerungstemperatur 60°C, (Vorwärtsprimer) 5'-CCAAGCGCAGCCTCTTCTTCAACC-3' (SEQ ID NO:12), (umgekehrter Primer) 5'-CCAGCAGCGTGGAGGCAGCATCTGCC-3' (SEQ ID NO:13); Sprecher et al. (1998) Arch. Dermatol. Res. 290, 656)
h2RFC2, Produktgröße 167 bp, 37 Zyklen, Anlagerungstemperatur 56°C, (Vorwärtsprimer) 5'-CCATCGCCACCTTTCAGATTGC-3' (SEQ ID NO:14), (umgekehrter Primer) 5'-CGGAGTATAACTGGAACTGCTTGCG-3' (SEQ ID NO:15).

Die Identität aller PCR-Produkte wurde durch anschließende Sequenzierung bestätigt.

### 5. Western Blot-Analyse

Die humanen Nabelschnurvenen-Endothelzellen wurden durch fünfmaliges aufeinanderfolgendes Einfrieren in flüssigem Stickstoff und Auftauen bei 37°C aufgeschlossen. Protein-Extrakte wurden wie bei Hecker et al. (1994) Biochem J. 299, 247 beschrieben hergestellt. 20-30 µg Protein wurden mit Hilfe einer 10%igen Polyacrylamid-Gelelektrophorese unter denaturierenden Bedingungen in der Gegenwart von SDS nach Standardprotokoll aufgetrennt und auf eine BioTrace™ Polyvinylidene Fluoride Transfermembran (Pall Corporation, Roßdorf, Deutschland) transferiert. Zum immunologischen Nachweis von Caveolin-1 wurde ein polyklonaler primärer Anti-Human-Antikörper von BD Biosciences, Heidelberg, Deutschland verwendet. Für den Nachweis des hFRC-Proteins diente ein polyklonaler Anti-Human-Antikörper (freundlicherweise zur Verfügung gestellt von Dr. Hamid M. Said, Veterans Affairs Medical Center, Long Beach, California, USA). CD40-Protein wurde mit einem polyklonalen Anti-Human-Antikörper (Research Diagnostics Inc., Flanders, New Jersey, USA) detektiert. Die Proteinbanden wurden nach Hinzufügen eines Peroxidase-gekoppelten Anti-Maus-IgG bzw. Anti-Kaninchen-IgG (1:3000, Sigma, Deisenhofen, Deutschland) mit Hilfe der Chemilumineszenz-Methode (SuperSignal Chemiluminescent Substrate; Pierce Chemical, Rockford, IL, USA) und nachfolgender Autoradiographie (Hyperfilm™ MP, Amersham Pharmacia Biotech, Buckinghamshire, England) visualisiert. Der Auftrag und Transfer gleicher Proteinmengen wurde nach "Strippen" der Transfermembran (5 Minuten 0.2 N NaOH, nachfolgend 3 x 10 Minuten Waschen mit H₂O) durch Nachweis gleicher Proteinbanden von β-Actin mit einem monoklonalen primären Antikörper und einem Peroxidase-gekoppelten Anti-Maus IgG (beide von Sigma-Aldrich, 1:3000 Verdünnung) gezeigt.

### 6. Fluoreszenz-Mikroskopie

Vor Versuchsbeginn wurden die in 24-Loch Zellkulturplatten kultivierten Endothelzellen einmal mit 37°C warmer Ringerlösung gewaschen (Zusammensetzung: 145 mmol/l Na⁺, 5 mmol/l K⁺, 156 mmol/l Cl⁻, 2 mmol/l Ca²⁺, 1 mmol/l Mg²⁺, 10 mmol/l Hepes, 10 mmol/l D-Glucose, pH 7,35). Danach wurden je nach Versuchsansatz 150 µl 37°C warme modifizierte bzw. nichtmodifizierte Ringerlösung auf die Zellen appliziert und das FITC-markierte Decoy-Oligonukleotid in einer finalen Konzentration von 10 µmol/l hinzugefügt. Nach einer Inkubationszeit von bis zu 180 min bei 37°C und Raumluft wurden die Zellen dreimal mit 1 ml warmer nichtmodifizierter Ringerlösung gewaschen. Die Fluoreszenzintensitäten wurden mit einer MicroMax CCD-Kamera (Princeton Instruments Inc., Trenton, NJ, USA), die an ein Axiovert S100 TV Mikroskop (Zeiss, Göttingen, Deutschland) gekoppelt war, bei einer Anregungswellenlänge von 494 nm, einer Emissionswellenlänge von 518 nm und 200facher Vergrößerung dokumentiert. Die Fluoreszenzaufnahmen (je Teilversuchsansatz wurde eine Aufnahme gemacht) und die folgende Quantifizierung wurden mit Hilfe der MetaMorph V3.0 Software (Universal Imaging, West Chester, PA, USA) durchgeführt. Für die Quantifizierung wurden sämtliche Fluoreszenz-Aufnahmen eines Versuchsansatzes zunächst auf die gleiche Helligkeits- und Kontraststufe angeglichen. Anschließend konnte mit Hilfe der Software eine über die einzelnen Bildpunkte integrierte Gesamthelligkeit eines jeden Bildes als Maß für die Fluoreszenz-Intensität stellvertretend für die intrazelluläre Konzentration des Decoy-Oligonukleotids ermittelt werden.

### 7. Statistische Analyse

Wenn nicht anders angezeigt, sind alle Daten in den Figuren als Mittelwert ± SEM von n Experimenten angegeben. Die statistische Auswertung wurde mittels einseitiger Varianzanalyse (ANOVA) gefolgt von einem Dunnett Post-Test durchgeführt. Ein P-Wert <0.05 wurde als statistisch signifikanter Unterschied angesehen.

## Patentansprüche

1. Pharmazeutische Formulierung umfassend Nukleinsäuren, **dadurch gekennzeichnet, dass** die Formulierung
a) einen pH-Wert im Bereich von pH 6,2 bis pH 7,0 und eine Chloridionen-Konzentration im Bereich von 5 bis 100 mmol/l aufweist, oder
b) einen pH-Wert im Bereich von pH 6,2 bis pH 7,0 und nichtsteroidale Antiphlogistika mit einer Konzentration im Bereich von 10 bis 500 µmol/l aufweist, oder
c) eine Chloridionen-Konzentration im Bereich von 5 bis 100 mmol/l und nichtsteroidale Antiphlogistika mit einer Konzentration im Bereich von 10 bis 500 µmol/l aufweist.

2. Formulierung nach Anspruch 1, wobei die Formulierung einen pH-Wert im Bereich von pH 6,2 bis pH 7,0, eine Chloridionen-Konzentration im Bereich von 5 bis 100 mmol/l und nichtsteroidale Antiphlogistika mit einer Konzentration im Bereich von 10 bis 500 µmol/l aufweist.

3. Formulierung nach einem der vorhergehenden Ansprüche, wobei der pH-Wert 6,5 oder 7,0 ist.

4. Formulierung nach einem der vorhergehenden Ansprüche, wobei die Chloridionen eine Konzentration im Bereich von 5 bis 50 mmol/l aufweisen.

5. Formulierung nach einem der vorhergehenden Ansprüche, wobei die Chloridionen eine Konzentration im Bereich von 5 bis 10 mmol/l aufweisen.

6. Formulierung nach einem der vorhergehenden Ansprüche, wobei die nichtsteroidalen Antiphlogistika eine Konzentration im Bereich von 50 bis 250 µmol/l aufweisen.

7. Formulierung nach einem der vorhergehenden Ansprüche, wobei die nichtsteroidalen Antiphlogistika eine Konzentration von 100 µmol/l aufweisen.

8. Formulierung nach einem der vorhergehenden Ansprüche, wobei die nichtsteroidalen Antiphlogistika Flurbiprofen oder Indoprofen sind.

9. Formulierung nach einem der vorhergehenden Ansprüche, ferner umfassend Trägerstoffe und Zusatzstoffe.

## Claims

1. Pharmaceutical formulation comprising nucleic acids, **characterized in that** the formulation
a) has a pH-value in the range of pH 6.2 to pH 7.0 and a chloride ion concentration in the range of 5 to 100 mmol/l, or
b) has a pH-value in the range of pH 6.2 to pH 7.0 and non-steroidal antiphlogistics with a concentration in the range of 10 to 500 µmol/l, or
c) has a chloride ion concentration in the range of 5 to 100 mmol/l and non-steroidal antiphlogistics with a concentration in the range of 10 to 500 µmol/l.

2. Formulation of claim 1, wherein the formulation has a pH-value in the range of pH 6.2 to pH 7.0, a chloride ion concentration in the range of 5 to 100 mmol/l and non-steroidal antiphlogistics with a concentration in the range of 10 to 500 µmol/l.

3. Formulation of any one of the preceding claims, wherein the pH-value is 6.5 or 7.0.

4. Formulation of any one of the preceding claims, wherein the chloride ions have a concentration in the range of 5 to 50 mmol/l.

5. Formulation of any one of the preceding claims, wherein the chloride ions have a concentration in the range of 5 to 10 mmol/l.

6. Formulation of any one of the preceding claims, wherein the non-steroidal antiphlogistics have a concentration in the range of 50 to 250 µmol/l.

7. Formulation of any one of the preceding claims, wherein the non-steroidal antiphlogistics have a concentration of 100 µmol/l.

8. Formulation of any one of the preceding claims, wherein the non-steroidal antiphlogistics are Flurbiprofen or Indoprofen.

9. Formulation of any one of the preceding claims, further comprising carriers and additives.

## Revendications

1. Formulation pharmaceutique comprenant des acides nucléiques, **caractérisée en ce que** la formulation présente
a) une valeur de pH dans la plage de pH 6,2 à pH 7,0 et une concentration en ions chlorures dans la plage de 5 à 100 mmoles/l, ou
b) une valeur de pH dans la plage de pH 6,2 à pH 7,0 et des anti-inflammatoires non stéroïdiens à une concentration dans la plage de 10 à 500 µmoles/l, ou
c) une concentration en ions chlorures dans la plage de 5 à 100 mmoles/l et des anti-inflammatoires non stéroïdiens à une concentration dans la plage de 10 à 500 µmoles/l.

2. Formulation selon la revendication 1, dans laquelle la formulation présente une valeur de pH dans la plage de pH 6,2 à pH 7,0, une concentration en ions chlorures dans la plage de 5 à 100 mmoles/l et des anti-inflammatoires non stéroïdiens à une concentration dans la plage de 10 à 500 µmoles/l.

3. Formulation selon l'une quelconque des revendications précédentes, dans laquelle la valeur de pH est de 6,5 ou 7,0.

4. Formulation selon l'une quelconque des revendications précédentes, dans laquelle les ions chlorures sont présents en une concentration dans la plage de 5 à 50 mmoles/l.

5. Formulation selon l'une quelconque des revendications précédentes, dans laquelle les ions chlorures sont présents en une concentration dans la plage de 5 à 10 mmoles/l.

6. Formulation selon l'une quelconque des revendications précédentes, dans laquelle les anti-inflammatoires non stéroïdiens sont présents en une concentration dans la plage de 50 à 250 µmoles/l.

7. Formulation selon l'une quelconque des revendications précédentes, dans laquelle les anti-inflammatoires non stéroïdiens sont présents en une concentration de 100 µmoles/l.

8. Formulation selon l'une quelconque des revendications précédentes, dans laquelle les anti-inflammatoires non stéroïdiens sont le flurbiprofène ou l'indoprofène.

9. Formulation selon l'une quelconque des revendications précédentes, comprenant en outre des véhicules et des additifs.


### SEQUENCE LISTING

<110> Avontec GmbH
<120> Formulierung zur Einschleusung von Nukleinsäuren in eukaryotische Zellen
<130> HEC-007 PCT
<140> unknown
   <141> 2003-09-02
<150> 102 40 418.6
   <151> 2002-09-02
<160> 15
<170> PatentIn version 3.1
<210> 1
   <211> 21
   <212> DNA
   <213> synthetic sequence
<220>
   <221> Decoy-Oligonukleotid
   <222> (1)..(21)
   <223>
<400> 1
   cgcttgatga ctcagccgga a 21
<210> 2
   <211> 20
   <212> DNA
   <213> synthetic sequence
<220>
   <221> Decoy-Oligonukleotid
   <222> (1)..(20)
   <223>
<400> 2
   tgcagattgc gcaatctgca 20
<210> 3
   <211> 25
   <212> DNA
   <213> synthetic sequence
<220>
   <221> Decoy-Oligonukleotid
   <222> (1)..(25)
   <223>
<400> 3
   catgttatgc atattcctgt aagtg 25
<210> 4
   <211> 17
   <212> DNA
   <213> synthetic sequence
<220>
   <221> Antisense-Oligonukleotid
   <222> (1) .. (17)
   <223>
<400> 4
   atgtccctcc gagtcta 17
<210> 5
   <211> 17
   <212> DNA
   <213> synthetic sequence
<220>
   <221> Antisense-Oligonukleotid
   <222> (1)..(17)
   <223>
<400> 5
   ctcgatcctg actactg 17
<210> 6
   <211> 17
   <212> DNA
   <213> synthetic sequence
<220>
   <221> Antisense-Oligonukleotid
   <222> (1)..(17)
   <223>
<400> 6
   caaaggtagc acacgag 17
<210> 7
   <211> 17
   <212> DNA
   <213> synthetic sequence
<220>
   <222> Antisense-Oligonukleotid
   <222> (1)..(17)
   <223>
<400> 7
   acatggacac gaagcag 17
<210> 8
   <211> 25
   <212> DNA
   <213> synthetic sequence
<220>
   <221> Primer
   <222> (1)..(25)
   <223>
<400> 8
   caaggtcagc aactacagcc gaggg 25
<210> 9
   <211> 24
   <212> DNA
   <213> synthetic sequence
<220>
   <221> Primer
   <222> (1) .. (24)
   <223>
<400> 9
   tgagcagcca cagcagcatt aggg 24
<210> 10
   <211> 24
   <212> DNA
   <213> synthetic sequence
<220>
   <221> Primer
   <222> (1) .. (24)
   <223>
<400> 10
   ctgtgtagcc accatgtgca gtgc 24
<210> 11
   <211> 22
   <212> DNA
   <213> synthetic sequence
<220>
   <221> Primer
   <222> (1)..(22)
   <223>
<400> 11
   tgtgacaatc ctcccaccag cg 22
<210> 12
   <211> 24
   <212> DNA
   <213> synthetic sequence
<220>
   <221> Primer
   <222> (1) .. (24)
   <223>
<400> 12
   ccaagcgcag cctcttcttc aacc 24
<210> 13
   <211> 26
   <212> DNA
   <213> synthetic sequence
<220>
   <221> Primer
   <222> (1) .. (26)
   <223>
<400> 13
   ccagcagcgt ggaggcagca tctgcc 26
<210> 14
   <211> 22
   <212> DNA
   <213> synthetic sequence
<220>
   <221> Primer
   <222> (1)..(22)
   <223>
<400> 14
   ccatcgccac ctttcagatt gc 22
<210> 15
   <211> 25
   <212> DNA
   <213> synthetic sequence
<220>
   <221> Primer
   <222> (1)..(25)
   <223>
<400> 15
   cggagtataa ctggaactgc ttgcg 25